# FASCICULE DE BREVET EUROPEEN

(11) **EP 3 297 733 B1**
(45) Date de publication et mention de la délivrance du brevet: **12.08.2026**
(21) Numéro de dépôt: 16726819.2
(22) Date de dépôt: 20.05.2016
(51) Int. Cl.: A61K 31/185, A61K 31/385, A61K 8/46, A23L 33/10, A23L 33/12, A23L 33/175, A61K 8/49, A61K 9/00, A61K 9/06, A61K 9/14, A61P 17/00, A61P 27/00, A61Q 19/00, A61P 27/02

(54) **COMPOSITION PHARMACEUTIQUE COMPRENANT UNE ASSOCIATION D'ACIDE LIPOIQUE ET DE TAURINE EN TANT QU'AGENT OSMOPROTECTEUR**
PHARMAZEUTISCHE ZUSAMMENSETZUNG DIE EINE KOMBINATION VON LIPONSÄURE UND TAURIN ALS OSMOPROTEKTIVES MITTEL UMFASST
PHARMACEUTICAL COMPOSITION COMPRISING A COMBINATION OF LIPOIC ACID AND TAURINE AS OSMOPROTECTIVE AGENT

(30) Priorité: 21.05.2015 FR 1554590; 18.01.2016 FR 1650372
(43) Date de publication de la demande: 28.03.2018
(73) Titulaire: DULCIS HEALTH SCIENCE, 98000 Monaco (MC)
(72) Inventeur: CLARET, Martine, 1025 Saint Sulpice (CH); CLARET, Claude, 1025 Saint Sulpice (CH); CHATARD-BAPTISTE, Caroline, 06100 Nice (FR)
(74) Mandataire: Be IP
(86) Numéro de dépôt international: PCT/EP2016/061358
(87) Numéro de publication internationale: WO 2016/184998

(56) Documents cités:
- EP-A2- 2 311 454
- EP-B1- 2 311 454
- WO-A1-2005/027950
- US-A- 5 817 630
- US-A1- 2001 031 744
- US-A1- 2006 127 505
- US-A1- 2012 258 168
- US-B1- 6 620 425
- US-B1- 6 649 195
- DATABASE WPI Week 201172, Derwent World Patents Index; AN 2011-L12576, XP002759503

## Description

### DOMAINE DE L'INVENTION

La présente invention concerne une composition pharmaceutique appropriée pour une application topique comprenant une association d'acide lipoïque et de taurine pour son utilisation en tant qu'agent osmoprotecteur pour la prévention et le traitement des affections de la peau et des muqueuses associées à un déséquilibre osmotique.

### ETAT DE LA TECHNIQUE

Un déséquilibre osmotique est observé dans les affections de la peau et des muqueuses des mammifères, en particuliers humains, généralement sous forme de sécheresse cutanée ou xérose, pouvant être le signe de dermatite atopique, ichtyose, eczéma, psoriasis ; ou sous forme de sécheresse oculaire, pouvant être le signe d'un dysfonctionnement des glandes de Meibomius, d'un syndrome hyperévaporatif, ou d'une insuffisance lacrymale, ainsi que sous forme d'œdème oculaire.

Les traitements usuels de ces affections de la peau ou des muqueuses vont de l'application de crèmes hydratantes, émollientes, voire de crèmes ou pommades à base de corticoïdes, voire des immunomodulateurs locaux.

La taurine est un acide aminosulfonique naturel produit par le pancréas des mammifères par transformation de la cystéine. Son usage est connu comme additif alimentaire dans des boissons énergisantes. L'usage de la taurine est aussi décrit dans des compositions ophtalmiques pour ses propriétés modulatrices des ions calciums dans les cellules, supposées prévenir la dégénérescence maculaire (US 2006/188492) ou comme agent chélatant (US 5 817 630).

L'acide lipoïque, également appelé acide thioctique (CAS n° 0001077-28-7) et ses dérivés sont connus pour leurs propriétés antioxydantes. Il est employé comme complément alimentaire sous forme de comprimés (Liponsäure-ratiopharm^{®} commercialisé en Allemagne par la société Ratiopharm) ou encore en injection sous forme de solutions diluées injectables (Neurium^{®} commercialisé en Allemagne par la société Hexal), éventuellement dans l'accompagnement de traitements de patients diabétiques. Des compositions ophtalmiques ou cosmétiques pouvant comprendre de l'acide lipoïque comme agent antioxydant sont décrites dans des demandes de brevet (US 2006/127505, US 2001/031744, US 5 817 630, US 2012/258168, US 6649195 B1, WO 2005/027950, US 6 620 425 B1, EP 2 311 454 A2, CN 102 144 780 A, US 2006/188492, US 2004/265345, US 5 817 630, WO 02098345, DE10229995, WO 01/93824, US 2005/192229, BR PI0 800 818, CN 103 860 625 et JP 2013 241398).

Ses propriétés antimicrobiennes ont été également décrites pour son utilisation dans des solutions de nettoyage de lentilles à base de BDT (US 6 162 393).

Les propriétés antioxydantes de l'acide lipoïque ont été également mises en avant pour étudier son efficacité sur la cataracte chez le rat diabétique (Masami Kojima & col., Japanese Journal of Ophthalmology, January 2007, Volume 51, Issue 1, pp 10-13).

Après avoir testé l'acide lipoïque sur des cellules de cornée, les inventeurs ont constaté que l'effet antioxydant observé est assez faible, plus faible que l'effet attendu au vu de ce qui est décrit pour des compléments alimentaires.

Ils ont maintenant trouvé que l'acide lipoïque avait des propriétés osmoprotectrices importantes et avantageuses qui permettaient son utilisation dans la prévention et le traitement de telles affections de la peau et des muqueuses, notamment de l'œil, en remplacement ou en complément des traitements usuels, sans rapport avec les affections supposées être traitées par les compositions comprenant de l'acide lipoïque de l'état de la technique. En particulier, ils ont pu constater que l'association de l'acide lipoïque avec la taurine, permet d'augmenter et de prolonger l'effet osmoprotecteur de cette combinaison par rapport aux effets observés séparément pour chaque constituant de l'association. L'acide lipoïque, agit rapidement après application alors que l'effet osmoprotecteur de la taurine apparaît et se prolonge lorsque celui de l'acide lipoïque commence à diminuer.

### EXPOSE DE L'INVENTION

La présente invention concerne une composition pharmaceutique appropriée pour une application topique comprenant une association d'acide lipoïque et de taurine, et un véhicule pharmaceutiquement acceptable pour son utilisation dans la prévention ou le traitement d'une affection de la peau dans laquelle apparait une sécheresse cutanée, l'affection de la peau étant choisie parmi la dermatite atopique, l'ichtyose, l'eczéma ou le psoriasis, ou d'une affection oculaire liée à la sécheresse oculaire, l'affection oculaire étant choisie parmi un dysfonctionnement des glandes Meibomius, un syndrome hyperévaporatif ou une insuffisance lacrymale, le rapport pondéral acide lipoïque / taurine allant de 0.004 à 0.05. Des modes de réalisations de l'invention sont exposées dans le jeu de revendications joint.

### DESCRIPTION DETAILLEE DE L'INVENTION

La présente invention concerne une composition pharmaceutique appropriée pour une application topique comprenant une association d'acide lipoïque et de taurine pour son utilisation en tant qu'agent osmoprotecteur pour la prévention et le traitement des affections de la peau et des muqueuses, notamment de l'oeil, associées à une sécheresse cutanée ou oculaire, le rapport pondéral acide lipoique/taurine allant de 0,004 à 0,05.

Par acide lipoïque, on entend selon l'invention l'acide 5-(1,2-dithiolan-3-yl)pentanoïque, sous forme racémique ou ses énantiomères en toutes proportions, en particulier l'énantiomère R, pur ou en mélange où la proportion d'énantiomère R est supérieure à celle de l'énantiomère S, et leurs sels pharmaceutiquement acceptables.

Parmi les sels pharmaceutiquement acceptables, on entend selon l'invention avantageusement les sels d'addition de l'acide lipoïque avec une base pharmaceutiquement acceptable, qu'il s'agisse d'une base organique notamment comprenant un groupe amino, comme l'ammoniac, la lysine, l'arginine et autres composés connus de la pharmacopée ou avec une base inorganique pharmaceutiquement acceptable telle que la soude, la potasse, l'hydroxyde de calcium et d'autres bases inorganiques connues de la pharmacopée. Préférentiellement, le sel pharmaceutiquement acceptable est un sel de métal alcalin (sodium, potassium), de métal alcalino-terreux (calcium, magnésium) ou un ion d'aluminium, plus préférentiellement un sel de sodium.

Selon un mode préférentiel de réalisation de l'invention, l'acide lipoïque employé dans la composition selon l'invention est un sel de l'énantiomère R de l'acide lipoïque, en particulier sel de sodium (CAS 176110-81-9) ou de magnésium.

Par taurine, on entend selon l'invention l'acide 2-aminoethanesulfonique, ses diastéréoisomères, purs ou leurs mélanges en toutes proportions et leurs sels pharmaceutiquement acceptables.

Elle peut être d'origine naturelle ou synthétique.

Les sels de taurine peuvent être des sels d'acides ou de bases. De manière avantageuse, la taurine employée dans la composition selon l'invention est un mélange de diastéréosiomères de l'acide 2-aminoethanesulfonique, en particulier obtenu par synthèse chimique.

La composition comprend de l'acide lipoïque et de la taurine dans un rapport pondéral acide lipoïque / taurine allant de 0,004 à 0,05, préférentiellement de environ 0,005 à environ 0,02, en particulier de environ 0,005, 0,01, 0,02 ou 0,04.

Les compositions topiques sont bien connues de l'homme du métier dans les domaines cosmétiques et pharmaceutiques.

Elles se présentent sous toutes formes usuelles connues de l'homme du métier pour une application topique, notamment sous forme de suspension ou de dispersion dans des solvants ou des corps gras, sous forme de dispersion vésiculaire ou encore sous forme d'émulsion telle qu'une crème ou un lait, sous forme de pommade, de gel, d'onguent, de bâtonnet solide, de mousse aérosol ou de spray.

Les compositions de l'invention peuvent comprendre les adjuvants usuels mis en œuvre pour la préparation de telles formulations, comme les corps gras, les solvants organiques, les tensioactifs ioniques zwitterioniques ou non-ioniques, les adoucissants, les antioxydants, les opacifiants, les stabilisants, les épaississants ioniques ou non ioniques, les silicones, les agents anti-mousse, les agents hydratants, les vitamines, les parfums, les conservateurs, les charges, les séquestrants, les colorants, les bases ou les acides nécessaires à la régulation du pH, ou tout autre ingrédient habituellement utilisé pour la préparation de compositions cosmétiques ou pharmaceutiques. Les compositions selon l'invention sont préparées selon les techniques bien connues de l'homme du métier.

Selon un mode de réalisation de l'invention, l'association d'acide lipoïque et de taurine est utilisée en association avec d'autres actifs, comme un autre agent osmoprotecteur, un agent anti-inflammatoire et/ou un agent anti-oxydant.

Parmi les autres agents osmoprotecteurs susceptibles d'être employés selon l'invention, on citera la glycérine, la L-carnitine, l'érythritol, le tréhalose, l'ectoïne, la bétaïne, la sarcosine, l'urée, de préférence la glycérine.

Parmi les agents à activité anti-inflammatoire susceptibles d'être employés avec l'acide lipoïque, on citera la dexaméthasone, le flurbiprofène, la fluorométholone, l'acide salicylique, l'hydrocortisone, la triamcinolone, la rimexolone de préférence le flurbiprofène et la dexaméthasone.

On citera également comme autres actifs les dérivés ou analogues de l'acide rétinoïque, en particulier le rétinol et ses dérivés, notamment ses esters, en particulier ses esters d'acides gras comme le rétinyl palmytate.

L'acide lipoïque et le ou les autres agents à activité anti-inflammatoire, en particulier l'hydrocortisone et la dexaméthasone peuvent être employés ensemble dans une même composition ou dans des compositions séparées.

Parmi les agents antioxydants susceptibles d'être employés avec l'acide lipoïque, on citera la vitamine E, le glutathion, la vitamine A, la vitamine C.

L'association d'acide lipoïque et de taurine et le ou les autres agents anti-oxydants peuvent être employés ensemble dans une même composition ou dans des compositions séparées. De manière avantageuse, le rapport acide lipoïque/ agent(s) anti-oxydant(s) appliqué dans une même composition ou dans des compositions séparées va de 0,001 à 0,01.

Selon un mode particulier de réalisation de l'invention, l'association d'acide lipoïque et de taurine est employée avec un autre un autre agent osmoprotecteur et/ou au moins un agent anti-inflammatoire et/ou au moins un agent anti-oxydant dans une même composition, de préférence dans les proportions décrites ci-dessus.

Selon un autre mode de réalisation de l'invention, la composition comprend également un polymère mucomimétique, en particulier de l'acide hyaluronique et ses sels.

La composition comprenant l'association d'acide lipoïque et de taurine selon l'invention pourra en outre comprendre des agents à action hydratante, cicatrisante, anti-inflammatoire, anti-âge, apaisante, anti-irritante, restructurante, émolliente, seuls ou en mélanges en toutes proportions.

Selon un mode particulier de réalisation de l'invention, la composition comprend également de l'acide hyaluronique ou ses sels, de préférence à une teneur allant de 0,05 à 2 % en poids par rapport au poids total de la composition. Les différentes formes d'acide hyaluronique et ses sels employés dans des compositions pharmaceutiques ou cosmétiques sont bien connues de l'homme du métier et susceptibles d'être employées dans les compositions selon l'invention. On citera notamment le hyaluronate de sodium avec une viscosité intrinsèque allant de 1 à 2,5 m3/kg.

Selon un mode préféré de réalisation de l'invention, la composition contenant l'association d'acide lipoïque et de taurine selon l'invention est une composition sans conservateurs.

Les agents conservateurs généralement employés dans des compositions topiques (cosmétiques, pharmaceutiques, ophtalmiques, etc.) pour éviter leur contamination par des germes, sont bien connus de l'homme du métier, comme les ammoniums quaternaires, notamment le chlorure de benzalkonium, l'alkyl-diméthyl-benzylammonium, le cétrimide, le chlorure de cétylpyridinium, le bromure de benzododécinium, le chlorure de benzothonium, le chlorure de cetalkonium, les conservateurs mercuriels, comme le nitrate/acétate/borate phénylmercurique, le thiomersal, les conservateurs alcooliques, comme le chlorobutanol, l'alcool benzylique, le phényléthanol, l'alcool phénylethylique, les acides carboxyliques, tels que l'acide sorbique, les phénols, en particulier méthyl/propyl parabène, les amidines, par exemple le chlorhexidine digluconate et/ou des agents chélateur comme l'EDTA seul ou en association avec au moins un autre conservateur.

Par « sans conservateurs », on entend selon l'invention une composition substantiellement dépourvue de tels conservateurs pour répondre à une indication « sans conservateurs ». Sa teneur en conservateurs est inférieure ou égale à 10 ppm, plus particulièrement inférieure ou égale à 1 ppm, préférentiellement égale à 0 ppm, aucun conservateur n'entrant dans sa composition.

En l'absence de conservateurs, la composition doit subir un traitement particulier au cours de sa préparation et de son conditionnement de manière à éviter et prévenir la contamination par des pathogènes. Ces traitements et procédures sont bien connus de l'homme du métier. En ce sens, une composition sans conservateurs selon l'invention se distingue d'une simple composition comprenant les mêmes ingrédients obtenue sans montrer de précautions particulières ni décrivant d'étapes du procédé permettant d'obtenir cette stérilité caractéristique des compositions selon l'invention, en particulier des compositions ophtalmiques.

Selon un premier mode particulier de réalisation de l'invention, l'association d'acide lipoïque et de taurine est utilisée sous la forme d'une composition topique appropriée pour son application sur la peau, particulièrement choisie parmi les crèmes, onguents, gels, pommades, solutions , lotions et émulsions.

L'association d'acide lipoïque et de taurine est avantageusement utilisée en tant qu'agent osmoprotecteur pour la prévention et le traitement d'une affection de la peau dans laquelle apparait une sécheresse cutanée, notamment dermatite atopique, ichtyose, eczéma, psoriasis.

L'homme du métier sait préparer des compositions pharmaceutiques ou cosmétiques topiques sous ces différentes formes, qu'il s'agisse d'émulsions eau dans huile ou huile dans eau, de solutions, de suspensions ou de gels. Les constituants de ces compositions et les proportions dans lesquelles ils sont employés sont bien connus de l'homme du métier, décrits notamment dans la Pharmacopée française.

De manière avantageuse, les compositions topiques selon l'invention sont dépourvues d'agents conservateurs.

Dans ces compositions topiques pour leur utilisation dans la prévention ou le traitement d'une affection de la peau dans laquelle apparait une sécheresse cutanée, la teneur en acide lipoïque est avantageusement de 0,0001 à 0,1 % en poids par rapport au poids total de la composition, de préférence de 0,001 à 0,05 %, en particulier environ 0,01 %.

La teneur en taurine est avantageusement de 0,001 % à 1,0 % en poids par rapport au poids total de la composition, de préférence de 0,01 à 0,1 %, plus préférentiellement d'environ 0,05 %.

Le rapport pondéral acide lipoïque / taurine dans une composition pharmaceutique est de 0,004 à 0,05, en particulier de 0,01 à 0,05, plus préférentiellement d'environ 0,02.

De manière avantageuse, la teneur en matière sèche est comprise entre 0.001 et 0.05% en poids. La matière sèche est constituée par l'ensemble des constituants entrant dans la composition de la formulation, à l'exception de l'eau.

Selon un autre mode de réalisation, la composition comprenant l'association d'acide lipoïque et de taurine est une composition ophtalmique (solution, gel, émulsion, suspension). L'invention concerne aussi cette composition ophtalmique comprenant l'association d'acide lipoïque et de taurine pour son utilisation pour la prévention et le traitement des affections oculaires liées à la sécheresse oculaire, pouvant être le signe d'un dysfonctionnement des glandes de Meibomius, d'un syndrome hyperévaporatif, ou d'une insuffisance lacrymale, et son utilisation dans les œdèmes oculaires, notamment cornéens.

La composition comprenant l'association d'acide lipoïque et de taurine est avantageusement utilisée sous la forme d'une composition ophtalmique appropriée pour son application sur l'œil d'un sujet, humain ou animal, plus particulièrement humain. En conséquence, la composition ophtalmique doit répondre à des caractéristiques techniques spécifiques des compositions ophtalmiques, et plus particulièrement liées à la sélection de ses composants. Ces composants qui sont « ophtalmiquement acceptables » ne doivent pas, individuellement ou associés dans la composition, provoquer de réactions secondaires de l'œil en dehors de l'effet recherché par la composition et ses actifs. L'œil étant un organe particulièrement sensible à un stress environnemental, la composition ne doit pas provoquer d'irritations parasites ou de réactions de type allergiques au détriment recherché, plus particulièrement dans le cas de compositions ophtalmiques destinées à traiter une affection ophtalmique. Le choix des constituants de la composition est donc très important, qui distingue la composition ophtalmique d'une simple composition inadaptée pour un usage ophtalmique. L'homme du métier est à même de choisir lesdits composants et de différencier une composition ophtalmique d'une simple composition destinée à un autre usage.

La composition ophtalmique a de préférence un pH compris entre 5 et 7,5. Elle comprend donc généralement un tampon approprié pour un usage ophtalmique, connu de l'homme du métier. On citera en particulier le citrate trisodique dihydraté et l'acide citrique monohydraté employés seuls ou en mélange.

La composition ophtalmique doit également être stérile pour ne pas apporter de pathogènes susceptibles de se développer et entrainer des complications ophtalmiques. Par « stérile », on entend au sens de la présente invention l'absence de germes au sens de la Pharmacopée Européenne, 8ème édition (2014). De manière préférentielle, la composition ophtalmique qui comprend l'association d'acide lipoïque et de taurine selon l'invention est une composition sans conservateurs.

Les compositions ophtalmiques se présentent généralement sous forme de liquides, solutions ou émulsions, mais aussi sous forme de gels ou de pommades. La composition ophtalmique selon l'invention est de préférence une solution ou une émulsion liquide pour une application par instillation d'une ou de plusieurs gouttes dans l'œil. La viscosité de la composition liquide est néanmoins choisie de manière à permettre son maintien sur l'œil, en particulier sur la cornée pendant un temps suffisant pour lui permettre d'agir.

La composition ophtalmique selon l'invention a de préférence une viscosité allant de 5 à 100 centipoises. Cette viscosité est mesurée selon les préconisations de la Pharmacopée Européenne 2.2.10, avec un viscosimètre rotatif, à 25°C, et 100s-1. D'autres appareils de mesure et méthodes adaptés à la mesure de la viscosité de solutions sont connus de l'homme du métier et permettent d'obtenir des résultats similaires.

La viscosité de la composition ophtalmique selon l'invention dépend de sa forme (solution ou émulsion) et est adaptée par l'ajout d'agents de viscosité « ophtalmiquement acceptables ». L'homme du métier connait bien les agents de viscosité susceptibles d'être employés pour la préparation de compositions ophtalmiques et les quantités à employer pour obtenir la viscosité recherchée. On citera en particulier l'hydroxypropylméthylcellulose, l'hydroxyethylcellulose, la carboxymethylcellulose, carbomères, les gels agar, polyvinylpyrrolidone et l'alcool polyvinylique.

De manière préférentielle, la composition ophtalmique selon l'invention comprend de l'hydroxypropylméthylcellulose ou de la carboxymethylcellulose, préférentiellement à une teneur allant de 0,05 à 0,5 % en poids d'hydroxpropylmethylcellulose ou de carboxymethylcellulose, avantageusement de 0,1 à 0,4 % en poids, plus avantageusement de 0,2 à 0,3 % en poids, particulièrement environ 0,25 % en poids.

Sauf indication contraire, les pourcentages sont exprimés en poids par rapport au poids total de la composition.

La composition ophtalmique pour son utilisation dans la prévention ou le traitement d'une affection oculaire liée à la sécheresse oculaire comprenant de l'acide lipoïque selon l'invention comprend avantageusement de 0,0001 à 0,1 % en poids d'acide lipoïque par rapport au poids total de la composition, et préférentiellement d'au moins 0,001 %, plus préférentiellement d'environ 0,001 %.

La teneur en taurine est avantageusement de 0,01 % à 1,0 % en poids par rapport au poids total de la composition, de préférence de 0,01 à 0,05 %.

Le rapport pondéral acide lipoïque / taurine va de 0,004 à 0,05.

Parmi les compositions ophtalmiques préférées selon l'invention on citera les compositions ci-après.

Selon un mode préféré de réalisation de l'invention, la composition ophtalmique comprenant de l'acide lipoïque selon l'invention est une émulsion huile dans eau.

La quantité appropriée de composition comprenant l'association d'acide lipoïque et de taurine appliquée dépendra de l'affection traitée, mais aussi de sa sévérité. L'âge du patient ou sa corpulence peuvent aussi influencer le praticien dans le choix de la dose à appliquer.

L'application de la dose appropriée peut être faite en une application, ou en plusieurs applications quotidiennes, pendant le temps nécessaire à l'obtention de l'effet recherché, c'est à dire la prévention ou le traitement des affections de la peau et des muqueuses, en particulier de l'oeil, associées à déséquilibre osmotique.

Dans le cas d'une composition ophtalmique liquide à instiller, la quantité appropriée sera donnée en nombre de gouttes à instiller.

### DESCRIPTION DES FIGURES

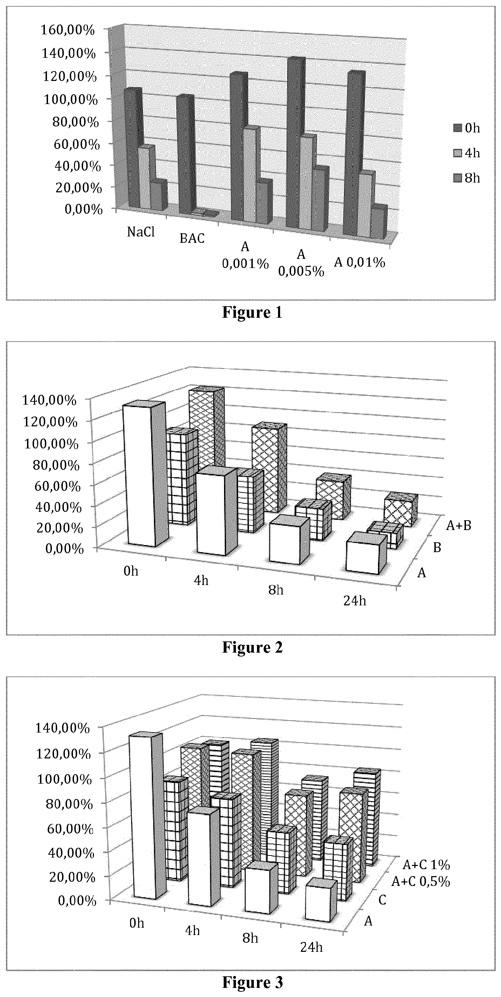

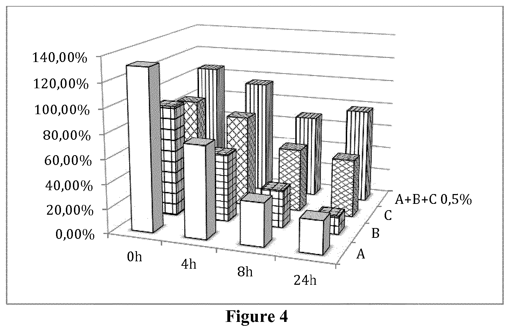

Les figures 1 à 4 représentent les résultats de viabilité cellulaire après stress osmotique pour différentes concentrations d'acide lipoïque, seul (comparatif) ou associé à de la glycérine (comparatif) et/ou de la taurine (invention).

### EXEMPLES

Les compositions données en exemples ci-après sont préparées selon les méthodes usuelles du domaine technique. Des précautions sanitaires particulières sont prises pour la préparation et le conditionnement des compositions sans conservateurs de manière à éviter les contaminations.

### Exemple 1 : Composition dermatologique (comparatif)

| **Composants** | **Quantité** |
|---|---|
| Caprylic/capric triglyceride | 20,00 % |
| Tribehenin PEG-20 Esters | 3,00 % |
| Niacinamide | 3,00 % |
| Taurine | 0.50 % |
| Glycerine | 0.50 % |
| Allantoïne | 0.20 % |
| Sodium lipoate | 0.01 % |
| Ceramide 3 | 0.10 % |
| Sodium Hyaluronate | 0.20 % |
| Sodium lipoate | 0.01 % |
| Hydroxyde de sodium (solution à 10%) | 1.50 % |
| Eau osmosée | Qsp 100% |

### Exemple 2 : Composition dermatologique (selon l'invention)

| **Composants** | **Quantité** |
|---|---|
| Cocamidopropyl Betaine | 10,00 % |
| Decyl glucoside | 5,00 % |
| Glycérine | 5,00 % |
| Niacinamide | 2,00 % |
| Citrate tri-sodium dihydrate | 1,45 % |
| Gomme de Xanthane | 0,75 % |
| Taurine | 0,50 % |
| Allantoïne | 0,20 % |
| Sodium hyaluronate | 0,10 % |
| acide citrique | 0,025 % |
| Acide lipoique | 0,005 % |
| Eau | Qsp 100% |

### Exemple 3 : Composition dermatologique (comparatif)

| **Composants** | **Quantité** |
|---|---|
| Caprylic/capric triglyceride | 25,00 % |
| Niacinamide | 4,00 % |
| Tribehenin PEG-20 Esters | 3,50 % |
| Glycerine | 3,00 % |
| Trisodium citrate | 1,45 % |
| Gomme de Xanthane | 0,80 % |
| Taurine | 0,50 % |
| Allantoïne | 0,50 % |
| Sodium Hyaluronate | 0,10 % |
| Ceramide NP | 0,10 % |
| Acide citrique | 0,05 % |
| Acide lipoique | 0,05 % |
| Eau | Qsp 100% |

### Exemple 4 : Composition dermatologique (selon l'invention)

| **Composants** | **Quantité** |
|---|---|
| Caprylic/capric triglyceride | 20,00 % |
| Tribehenin PEG-20 Esters | 3,00 % |
| Glycerine | 3,00 % |
| Trisodium citrate | 1,45 % |
| Gomme de Xanthane | 0,80 % |
| Sodium Hyaluronate | 0,50 % |
| Taurine | 0,50 % |
| Allantoïne | 0,20 % |
| Helianthus Annuus (huile de tournesol), Retinyl Palmitate | 0,10 % |
| Acide citrique | 0,05 % |
| Acide lipoique | 0,01 % |
| Eau | Qsp 100% |

### Exemple 5 : Composition ophtalmique (comparatif)

| **Composants** | **Quantité** |
|---|---|
| Hyaluronate de sodium | 0.20 % |
| Taurine | 0.5% |
| Lipoate sodium | 0.001% |
| Caprylic/Capric Triglyceride | 0,05% |
| Lécithine de soja | 0,15% |
| Carboxyméthylcellulose Sodique | 0,15% |
| Citrate trisodique dihydraté | 0.05% |
| Acide citrique | 0.02% |
| NaOH (1 N) | Qsp pH 6.7 |
| Chlorure de sodium | Qsp 150mOsmol/L |
| Eau ppi | Qsp 100 % |

### Exemple 6 : Composition ophtalmique (selon l'invention)

| **Composants** | **Quantité** |
|---|---|
| Chlorure de sodium | 5.00 % |
| Hyaluronate de sodium | 0.18 % |
| Taurine | 0.1% |
| Acide lipoïque | 0.001 % |
| Carboxyméthylcellulose Sodique | 0.3 % |
| Citrate disodique dihydraté | 1.5 % |
| Acide citrique monohydraté | Qsp pH 7 |
| Eau purifiée | Qsp 100 % |

### Exemple 7 : Activité osmoprotectrice de l'acide lipoïque et de la taurine seuls ou en mélanges

Une étude a été conduite afin de déterminer la capacité osmoprotectrice du lipoate de sodium (A) lors d'un stress hyperosmolaire sur des cellules humaines conjonctivales (WKD) et cornéennes (HCE).

Dans un deuxième temps, des tests ont été menés pour comparer l'effet de l'acide lipoïque, de la glycérine (B), et de la taurine (C), isolément et lorsque les molécules étaient combinées, sur les cellules HCE en conditions de stress hyperosmolaire.

L'activité osmoprotectrice de A, puis A avec B et C, a été évaluée en mesurant notamment la viabilité cellulaire. A cet effet, les cellules ont été pré-incubées pendant 17h avec la ou les substances test. Ensuite, le milieu a été retiré et les cellules ont été soumises à un stress hyperosmolaire par ajout de chlorure de sodium (100 mM NaCl) dans le milieu de culture (M199 pour cellules WKD et KSFM pour les cellules HCE). Les cellules du contrôle de pousse cellulaire étaient mises en contact avec du milieu isotonique. La viabilité cellulaire a été analysée avant le stress osmotique (0h) pour mesurer l'effet des molécules restées en contact avec les cellules pendant les 17h d'incubation, puis à 4h, 8h et 24h après l'induction du stress osmotique. La mesure de la viabilité cellulaire était réalisée via un test XTT.

Différentes concentrations de A ont été testées pour les essais "molécule seule": 0.0005%, 0.001%, 0.005%, 0.01% and 0.05% m/v. Pour les essais de mélange de molécule, les concentrations suivantes ont été testées : pour A : 0.001%, 0.005%, et 0.01 % m/v; pour B : 0.25% m/v ; et pour C : 0.5% and 1% m/v.

Pour s'assurer de la validité et de la significativité des données, les résultats obtenus ont été analysés statistiquement. Les résultats présentés ci-dessous résultent de la moyenne de 3 répétitions indépendantes, réalisées à des jours différents.

### Mesures de la viabilité cellulaire pour différentes concentrations en acide lipoïque (lipoate de sodium) avec des cellules WKD

| | **0h** | **4h** | **8h** | **24h** |
|---|---|---|---|---|
| **A 0,0005%** | 99,90% | 62,70% | 38,50% | 18,80% |
| **A 0,001%** | 98,50% | 69,60% | 42,40% | 18,50% |
| **A 0,005%** | 102,80% | 109,40% | 76,10% | 12,60% |
| **A 0,01%** | 98,10% | 119,90% | 82,90% | 8,90% |
| **A 0,05%** | 56,30% | 46,00% | 27,80% | 2,70% |
| **Nacl** | 98,80% | 50,70% | 33,50% | 25,80% |
| **Hidc** | 92,30% | 68,80% | 50,40% | 56,40% |

Les mesures de la viabilité cellulaire pour différentes concentrations en acide lipoïque (lipoate de sodium) avec des cellules HCE sont représentées sur la figure 1.

L'acide lipoïque présente une forte activité osmoprotectrice contre le stress osmotique à 0.005% et 0.001% (m/v) à 4h et 8h après le stress.

Les figures 2 à 4 représentent les résultats de viabilité obtenus avec différents mélanges de A avec B, C et B+C.

On observe une amélioration de l'effet osmoprotecteur de l'acide lipoïque lorsqu'il est associé à la glycérine (B) et à la taurine (C) ou les deux. Cette amélioration se traduit par une viabilité cellulaire supérieure à celle observée avec l'acide lipoïque seul, la glycérine seule ou la taurine seule. Elle est particulièrement marquée pour l'association acide lipoïque + taurine (figure 3), qui augmente cette action osmoprotectrice dans la durée, jusqu'à 24h après le stress.

### Exemple 8 : Activité osmoprotectrice de mélanges d'acide lipoïque et de taurine

Le protocole de l'exemple 7 est repris avec les 11 mélanges M1 à M11 de lipoate de sodium (A) et de taurine (C). Les résultats viabilité cellulaire obtenus confirment l'augmentation de l'action osmoprotectrice dans la durée observée pour les mélanges de l'exemple 7.

### Mesures de la viabilité cellulaire pour différentes concentrations en acide lipoïque (lipoate de sodium - (A) et de taurine (C) avec des cellules WKD

| | **A%** | **C%** | **A/C** | **t=0h** | **t=4h** | **t=8h** | **t=24h** |
|---|---|---|---|---|---|---|---|
| **M1** | 0,0001 | 0,10 | 0,001 | 81 % | 63 % | 50 % | 33 % |
| **M2** | 0,0002 | 0,10 | 0,002 | 84 % | 66 % | 51 % | 32 % |
| **M3** | 0,0050 | 1,00 | 0,005 | 84 % | 70 % | 67 % | 54 % |
| **M4** | 0,0050 | 0,50 | 0,010 | 93 % | 72 % | 66 % | 52 % |
| **M5** | 0,0050 | 0,25 | 0,020 | 96 % | 78 % | 65 % | 46 % |
| **M6** | 0,0100 | 0,50 | 0,020 | 96 % | 73 % | 69 % | 51 % |
| **M7** | 0,0200 | 1,00 | 0,020 | 82 % | 63 % | 58 % | 50 % |
| **M8** | 0,0200 | 1,00 | 0,020 | 90 % | 64 % | 57 % | 40 % |
| **M9** | 0,0050 | 0,10 | 0,050 | 92 % | 74 % | 58 % | 36 % |
| **M10** | 0,0500 | 0,25 | 0,200 | 61 % | 49 % | 40 % | 21 % |
| **M11** | 0,0500 | 0,10 | 0,500 | 73 % | 52 % | 27 % | 19 % |
| **Contrôle NaCl** | | | | 94 % | 68 % | 46 % | 22 % |

L'effet d'amélioration de l'osmoprotection apparaît encore plus marqué pour le rapport pondéral acide lipoïque / taurine de 0,004 à 0,05 (mélanges M3 à M9 selon l'invention). M1 et M2 sont des comparatifs.

### REFERENCES

US 5 817 630
US 6 162 393
US 2004/265345
US 2005/192229
US 2006/188492
WO 01/93824
WO 02098345
DE10229995
BR PI0 800 818
CN 103 860 625
JP 2013 241398
Masami Kojima & col., Japanese Journal of Ophthalmology, January 2007, Volume 51, Issue 1, pp 10-13

## Revendications

1. Composition pharmaceutique appropriée pour une application topique comprenant une association d'acide lipoïque et de taurine, et un véhicule pharmaceutiquement acceptable pour son utilisation dans la prévention ou le traitement d'une affection de la peau dans laquelle apparait une sécheresse cutanée, l'affection de la peau étant choisie parmi la dermatite atopique, l'ichtyose, l'eczéma ou le psoriasis, ou d'une affection oculaire liée à la sécheresse oculaire, l'affection oculaire étant choisie parmi un dysfonctionnement des glandes Meibomius, un syndrome hyperévaporatif ou une insuffisance lacrymale, **caractérisée en ce que** le rapport pondéral acide lipoïque / taurine va de 0,004 à 0,05.

2. Composition pour son utilisation selon la revendication 1 dans la prévention ou le traitement d'une affection de la peau dans laquelle apparait une sécheresse cutanée, **caractérisée en ce qu'**elle est appropriée pour une application sur la peau, choisie parmi les crèmes, les onguents, les gels, les lotions, les solutions, les émulsions et les pommades.

3. Composition pour son utilisation selon la revendication 2 dans la prévention ou le traitement d'une affection de la peau dans laquelle apparait une sécheresse cutanée, **caractérisée en ce qu'**elle comprend de 0,0001 à 0,1 % en poids d'acide lipoïque par rapport au poids total de la composition.

4. Composition pour son utilisation selon l'une des revendications 2 ou 3 dans la prévention ou le traitement d'une affection de la peau dans laquelle apparait une sécheresse cutanée, **caractérisée en ce qu'**elle comprend de 0,001 à 1,0 % en poids de taurine par rapport au poids total de la composition.

5. Composition ophtalmique pour son utilisation selon la revendication 1 dans la prévention ou le traitement d'une affection oculaire liée à la sécheresse oculaire, **caractérisée en ce qu'**elle comprend de 0,0001 à 0,1 % en poids d'acide lipoïque par rapport au poids total de la composition.

6. Composition ophtalmique pour son utilisation selon l'une des revendications 1 ou 5 dans la prévention ou le traitement d'une affection oculaire liée à la sécheresse oculaire, **caractérisée en ce qu'**elle comprend de 0,01 à 1,0 % en poids de taurine par rapport au poids total de la composition.

7. Composition pour son utilisation selon l'une des revendications 1, 5 ou 6 pour son utilisation dans les œdèmes oculaires, notamment cornéens.

## Patentansprüche

1. Pharmazeutische Zusammensetzung, die sich für eine topische Anwendung eignet, umfassend eine Kombination von Liponsäure und Taurin und einen pharmazeutisch akzeptablen Trägerstoff, für ihre Verwendung bei der Prävention oder der Behandlung einer Erkrankung der Haut, bei der eine Hauttrockenheit auftritt, wobei die Erkrankung der Haut ausgewählt ist aus atopischer Dermatitis, Ichthyose, Ekzem oder Psoriasis, oder einer Augenerkrankung, die mit Augentrockenheit verbunden ist, wobei die Augenerkrankung ausgewählt ist aus einer Funktionsstörung der Meibom-Drüsen, einem hyperevaporativen Syndrom oder einer Träneninsuffizienz, **dadurch gekennzeichnet, dass** das Gewichtsverhältnis Liponsäure:Taurin von 0,004 bis 0,05 beträgt.

2. Zusammensetzung zur Verwendung nach Anspruch 1 bei der Prävention oder der Behandlung einer Hauterkrankung, bei der eine Hauttrockenheit auftritt, **dadurch gekennzeichnet, dass** sie sich für eine Anwendung auf der Haut eignet, die ausgewählt ist aus Cremes, Salben, Gels, Lotionen, Lösungen, Emulsionen und Pomaden.

3. Zusammensetzung zur Verwendung nach Anspruch 2 bei der Prävention oder der Behandlung einer Hauterkrankung, bei der eine Hauttrockenheit auftritt, **dadurch gekennzeichnet, dass** sie, bezogen auf das Gesamtgewicht der Zusammensetzung, von 0,0001 bis 0,1 Gewichts-% Liponsäure umfasst.

4. Zusammensetzung zur Verwendung nach einem der Ansprüche 2 oder 3 bei der Prävention oder der Behandlung einer Hauterkrankung, bei der eine Hauttrockenheit auftritt, **dadurch gekennzeichnet, dass** sie, bezogen auf das Gesamtgewicht der Zusammensetzung, von 0,001 bis 1,0 Gewichts-% Taurin umfasst.

5. Ophthalmologische Zusammensetzung zur Verwendung nach Anspruch 1 bei der Prävention oder der Behandlung einer Augenerkrankung, die mit Hauttrockenheit verbunden ist, **dadurch gekennzeichnet, dass** sie, bezogen auf das Gesamtgewicht der Zusammensetzung, von 0,0001 bis 0,1 Gewichts-% Liponsäure umfasst.

6. Ophthalmologische Zusammensetzung zur Verwendung nach Anspruch 1 oder 5 bei der Prävention oder der Behandlung einer Augenerkrankung, die mit Hauttrockenheit verbunden ist, **dadurch gekennzeichnet, dass** sie, bezogen auf das Gesamtgewicht der Zusammensetzung, von 0,01 bis 1,0 Gewichts-% Taurin umfasst.

7. Zusammensetzung zur Verwendung nach einem der Ansprüche 1, 5 oder 6 zur Verwendung bei Augenödemen, insbesondere Hornhautödemen.

## Claims

1. Pharmaceutical composition suitable for topical application comprising an association of lipoic acid and taurine, and a pharmaceutically acceptable carrier for its use in the prevention or treatment of a skin condition in which cutaneous dryness occurs, said skin condition being chosen from atopic dermatitis, ichthyosis, eczema or psoriasis, or of an eye condition related to eye dryness, said eye condition being chosen from Meibomian gland dysfunction, hyper-evaporative syndrome or lacrimal insufficiency, **characterized in that** the weight ratio lipoic acid / taurine ranges from 0.004 to 0.05.

2. Composition for its use according to claim 1 in the prevention or treatment of a skin condition in which cutaneous dryness occurs, **characterized in that** it is suitable for application on the skin, chosen from creams, ointments, gels, lotions, solutions, emulsions and pomades.

3. Composition for its use according to claim 2 in the prevention or treatment of a skin condition in which cutaneous dryness occurs, **characterized in that** it comprises from 0.0001 to 0.1% by weight of lipoic acid relative to the total weight of the composition.

4. Composition for its use according to one of claims 2 or 3 in the prevention or treatment of a skin in which cutaneous dryness condition appears, **characterized in that** it comprises from 0.001 to 1.0% by weight of taurine relative to the total weight of the composition.

5. Ophthalmic composition for its use according to claim 1 in the prevention or treatment of an eye condition associated with eye dryness, **characterized in that** it comprises from 0.0001 to 0.1% by weight of lipoic acid relative to the total weight of the composition.

6. Ophthalmic composition for its use according to one of claims 1 or 5 in the prevention or treatment of an eye condition associated with eye dryness, **characterized in that** it comprises from 0.01 to 1.0% by weight of taurine relative to the total weight of the composition.

7. Composition for its use according to one of claims 1, 5 or 6 for use in ocular edema, particularly corneal edema.
